# EUROPÄISCHE PATENTSCHRIFT

(11) **EP 0 568 081 B1**
(45) Veröffentlichungstag und Bekanntmachung des Hinweises auf die Patenterteilung: **26.06.1996**
(21) Anmeldenummer: 93107005.6
(22) Anmeldetag: 29.04.1993
(51) Int. Cl.: A61B 3/18

(54) **Einrichtung zur Untersuchung bzw. Behandlung der Augen**
Ophthalmological apparatus for examination and/or treatment
Appareil ophthalmologique destiné à l'examen et/ou au traitement

(30) Priorität: 30.04.1992 DE 4214426
(43) Veröffentlichungstag der Anmeldung: 03.11.1993
(73) Patentinhaber: G. RODENSTOCK INSTRUMENTE GMBH, D-85521 Ottobrunn (DE)
(72) Erfinder: Wutz, Johann, W-8000 München 80 (DE); Zettel, Hans D., Dr., W-7050 Waiblingen (DE); Dolabdjian, Haig, Dr., W-8152 Feldkirchen/Westerham (DE); Müllerwiebus, Ernst, Dr., D-85521 Ottobrunn (DE); Karl, Hans-Peter, W-8000 München 71 (DE); Koch, Raluca, W-8000 München 80 (DE); Wilms, Karl-Heinz, W-8089 Emmering (DE)
(74) Vertreter: Münich, Wilhelm, Dr.

(56) Entgegenhaltungen:
- EP-A- 0 463 533
- WO-A-87/05204
- DE-A- 3 444 580
- US-A- 4 861 156

## Beschreibung

Die Erfindung bezieht sich auf eine Einrichtung zur Untersuchung und/oder Behandlung der Augen gemäß dem Oberbegriff des Patentanspruchs 1.

Zur Untersuchung der Augen werden in der Regel mehrere Untersuchungsgeräte benötigt. Beispielsweise bei der Refraktionsbestimmung verwendet der Augenarzt oder der Augenoptiker häufig ein objektives Refraktions-Meßgerät, mit dem die Refraktion der untersuchten Person ohne deren Mithilfe bestimmbar ist, sowie einen sogenannten Phoropter, bei dem eine Reihe von Gläsern mit unterschiedlicher optischer Wirkung auf mehreren, hintereinander angebrachten sog. Recoss-Scheiben angeordnet sind.

Darüberhinaus sind gerade in augenärztlichen Praxen oder in Krankenhäusern Perimeter zur Gesichtsfeldprüfung sowie Geräte für weitere Untersuchungen oder Behandlungsvorgänge, wie beispielsweise Tonometer oder Lasergeräte etc. vorhanden.

Die vorgenannten Augenuntersuchungsgeräte, aber auch die weiteren, vorstehend nicht erwähnten bekannten Augenuntersuchungs- oder Behandlungsgeräte sind in den letzten Jahren verstärkt mit einer elektronischen Steuerung ausgestattet worden, die häufig einen Computer und insbesondere einen sogenannten "Personal-Computer" (Industrie-Standard-Computer) aufweist.

In der US-PS 4 861 156 wird beispielsweise eine Einrichtung zur Untersuchung der Augen beschrieben, die ein Augenuntersuchungsgerät und einen damit verbundenen Computer aufweist.

Nachdem zunächst sogenannte "Stand-Alone-Lösungen" auf den Markt gekommen sind, ist in jüngerer Vergangenheit vorgeschlagen worden, die einzelnen Geräte untereinander mit an sich bekannten Computer-Netzwerken zu "vernetzen", um die Geräte beispielsweise in eine Praxis-EDV einbinden zu können.

Dieser Vorschlag hat jedoch eine Reihe von Nachteilen:
Bei einer "Netzwerk-Lösung" wird davon ausgegangen, daß jedes Untersuchungsgerät über einen eigenen Computer verfügt, der zum einen für die Steuerung des Untersuchungsgeräts verantwortlich ist, bzw. mit einer getrennten Steuereinheit zusammenarbeitet und der zum anderen für den Datenaustausch im Netzwerk sorgt. In der Regel werden die verschiedenen Untersuchungsgeräte jedoch nicht gleichzeitig betrieben. In der Regel stehen die Untersuchungsgeräte nämlich nur einer einzigen Person zur Verfügung, die damit nacheinander an einer untersuchten Person die verschiedenen Untersuchungen vornimmt.

Damit ist der beim Stand der Technik getriebene Aufwand, der den gleichzeitigen Betrieb sämtlicher Geräte ermöglichen würde, in der Praxis nicht erforderlich.

Ein weiteres Problem ergibt sich aus folgender Tatsache:
Häufig sind die Untersuchungsgeräte auf einem verschiebbaren Tisch oder hängend an einem verschiebbaren oder schwenkbaren Träger befestigt. Damit ist es möglich, daß die Stühle sowohl der untersuchten Person als auch der Untersuchungsperson ortsfest angeordnet sind und zur Durchführung der verschiedenen Untersuchungsvorgänge lediglich die Geräte bewegt werden müssen. Wenn nun jedem Gerät ein eigener Computer zugeordnet ist, müssen auch die Computer mit Bildschirm und Tastatur mitbewegt werden, so daß die Trägervorrichtung für die Geräte naturgemäß größer und schwerer werden muß.

Letztlich ist durch den Einsatz mehrerer Computer häufig auch die Bedienungsführung der einzelnen Geräte unterschiedlich, so daß gerade ein Vertreter, der nur kurzfristig mit den Geräten arbeitet, sich mit der Bedienung nicht zurechtfindet. Hierbei ist zu berücksichtigen, daß viele Augenärzte oder Augenoptiker noch mit "mechanischen" bzw. "nur teilweise elektronisch gesteuerten" Geräten arbeiten, so daß sie lediglich mit deren Bedienfeld vertraut sind.

Der Erfindung liegt die Aufgabe zugrunde, eine Vorrichtung zur Untersuchung der Augen mit wenigstens einem Augenuntersuchungsgerät, wie einem Phoropter oder einem Refraktometer sowie ggf. Hilfsgeräten, wie einem Scheitelbrechwertmesser, und mit einem Computer derart weiterzubilden, daß zum einen die Bedienung des Geräts auch für Personen, die nur mit herkömmlichen Augenuntersuchungsgeräten und nicht mit computergesteuerten Augenuntersuchungsgeräten vertraut sind, erleichtert wird, und daß zum anderen der technische Aufwand beim Anschluß weiterer Augenuntersuchungsgeräte, die anstelle des bereits angeschlossenen Geräts betrieben werden sollen, vereinfacht wird.

Eine erfindungsgemäße Lösung dieser Aufgabe ist im Patentanspruch 1 angegeben. Weiterbildungen der Erfindung sind Gegenstand der Unteransprüche.

Die Erfindung geht von der Erkenntnis aus, daß es nicht erforderlich ist, für mehrere Geräte jeweils einen gesonderten Computer vorzusehen. Deshalb sind alle Geräte mit einem einzigen Computer über (jeweils) eine Standard-Schnittstelle, beispielsweise eine serielle Normschnittstelle - z.B. eine RS 232 C- oder eine RS 422-Schnittstelle - (Anspruch 5) verbunden. Damit kann - sieht man einmal von speziellen Multitasking-Betriebssystemen ab, deren Einsatz selbstverständlich ebenfalls im Rahmen der Erfindung liegt - der Computer zu jedem Zeitpunkt zwar nur ein einziges Gerät steuern, in der täglichen Praxis ist dies jedoch kein Nachteil, da - wie bereits ausgeführt - ein Augenarzt oder Augenoptiker mit den ihm zur Verfügung stehenden Geräten nacheinander eine Person untersucht, so daß immer nur ein Gerät betriebsbereit sein muß.

Da nur ein einziger Computer vorgesehen ist, ist es nicht erforderlich, den Computer während des Untersuchungsvorgangs gemeinsam mit dem jeweils ihm zugeordneten Gerät zu verschieben, vielmehr kann der Computer an der für die Untersuchungsperson günstigsten Stelle in einer sog. Combi-Einheit, die mehrere Gerät ortsveränderlich trägt, angeordnet werden. Damit wird der mechanische Aufbau der Einheit, in der die einzelnen Untersuchungsgeräte integriert sind, vereinfacht.

Zur Vereinfachung der Bedienung wird der Computer über eine an sich bekannte graphische Benutzeroberfläche gesteuert, die beispielsweise über eine Maus oder eine Rollkugel (Anspruch 2), über die Tastatur (Anspruch 3) oder einen Touch-Screen (Anspruch 4) gesteuert wird.

Um dem mit elektronisch gesteuerten Augenuntersuchungsgeräten nicht vertrauten Benutzer, der sich jedoch in der Bedienung herkömmlicher Augenuntersuchungsgeräte auskennt, die Arbeit zu erleichtern, ist als graphische Benutzeroberfläche ein Bedienfeld dargestellt, dessen Auswahlfelder den Tasten bzw. Bedienelementen herkömmlicher Augenuntersuchungsgeräte der jeweils angesteuerten Art nachempfunden ist.

Beispielsweise ist es bevorzugt, zur Steuerung eines Phoropters, der in herkömmlicher Ausführung eine Reihe von Drehknöpfen zur Bedienung der Recoss-Scheiben aufweist, auf denen die einzelnen, in den Strahlengang einschwenkbaren Linsen angeordnet sind, derartige Drehknöpfe auf den Bildschirm darzustellen. Der Benutzer fährt mit der Maus oder der Tastatur den dargestellten "Drehknopf an" und betätigt ihn über die Maustaste bzw. eine Eingabetaste. Entsprechend tippt er bei einem "touch-Screen" den jeweiligen "Drehknopf" an.

Bei Anwahl eines einen Drehknopf darstellenden Auswahlfeldes werden wie bei einem mechanischen Phoropter nacheinander die einzelnen Dioptriewerte, die auf der Recoss-Scheibe vorhanden sind, angewählt. Hierzu ist es lediglich erforderlich, die häufig in Phoroptern bereits vorhandene Steuerung für die Schrittmotoren, die die Recoss-Scheiben drehen, so auszulegen, daß sie über eine Norm-Schnittstelle angesteuert werden kann.

Ist der gewünschte Dioptrie-Wert erreicht, stoppt die Bedienungsperson die weitere Anwahl durch "Loslassen" einer Steuerungstaste bzw. durch Wegnehmen des Fingers bzw. eines elektronischen Griffels vom Touch-Screen.

Entsprechendes gilt selbstverständlich für die Nachbildung des Bedienfeldes eines herkömmlichen automatischen Refraktors, eines Scheitelbrechwertmesser, eines Perimeters oder dgl. Dabei können selbstverständlich anstelle von "Drehknöpfen" auch "Schiebeschalter", die beispielsweise mit der Maus verschoben werden, wie sie in Windows-Applikationen üblich sind, oder Druckschalter eingesetzt werden.

Da der Computer die Funktion des oder der mit ihm verbundenen Geräte nicht nur steuert, sondern auch die Daten von diesen Geräten empfängt, wird eine Weiterbildung der Erfindung durch die Übernahme beispielsweise der Refraktionsdaten in ein Patienten-Stammdaten- und/oder ein Abrechnungsprogramm (Anspruch 6) dargestellt. Diese Programme können zusätzlich - oder bei einem Multitasking-Betriebssystem bzw. Betriebssystem-Aufsatz gleichzeitig - mit den Steuerungsprogrammen geladen werden.

In größeren Praxen oder in Krankenhäusern, in denen eine Reihe von Behandlungszimmern mit jeweils einem vollständigen Gerätesatz in der erfindungsgemäßen Ausbildung vorhanden sind, können selbstverständlich die einzelnen Steuerungscomputer, von denen jeder wenigstens ein, in der Regel jedoch mehrere Untersuchungs- und/oder Behandlungsgeräte steuert, untereinander vernetzt und beispielsweise mit einem Zentral-Computer (Server) verbunden werden, in den zentral von den einzelnen Steuerungscomputern die erhaltenen Daten eingebbar sind.

Da die Realisierung der Erfindung mit den heute bekannten Mitteln, wie beispielsweise Schrittmotoren für die Steuerung der Recoss-Scheiben von Phoroptern, die über eine serielle Schnittstelle ansteuerbar sind, Personal-Computern mit graphischen Betriebssystemen wie beispielsweise Windows, OS/2 oder X Windows für Unix für einen auf dem einschlägigen Gebiet tätigen Durchschnittsfachmann ohne weiteres möglich ist, wird auf die detaillierte Beschreibung eines Ausführungsbeispiels verzichtet.

## Patentansprüche

1. Einrichtung zur Untersuchung und/oder Behandlung der Augen, mit
- wenigstens einem Augenuntersuchungs- oder Behandlungsgerät, wie einem Phoropter oder einem Refraktometer sowie gegebenenfalls Hilfsgeräten, wie einem Scheitelbrechwertmesser,
- einem Computer, in den die von dem oder den Augenuntersuchungsgeräten sowie den gegebenenfalls vorhandenen Hilfsgeräten ermittelten Daten eingebbar sind, wobei
- das oder die Geräte über Standard-Schnittstellen mit dem Computer verbunden sind, und wobei
- der Computer so ausgebildet ist, daß er nicht nur Daten von dem oder den Geräten empfängt, sondern auch ein Steuerungsprogramm aufweist, mit dem er die Funktion des oder der mit ihm verbundenen Geräte steuert,
**gekennzeichnet** durch folgende Merkmale:
- der Computer weist Mittel auf, die so ausgebildet sind, daß auf dem Bildschirm des Computers als graphische Benutzeroberfläche ein Bedienfeld des oder der jeweils benutzten Geräte dargestellt ist, dessen Auswahlfelder optisch den Bedienelementen des oder der mit dem Computer verbundenen Augenuntersuchungsgeräte herkömmlicher Art nachempfunden sind, und
- der Computer weist weitere Mittel auf zur Steuerung der Funktion des oder der Geräte von der Bedienungsperson durch Anwahl des jeweiligen Auswahlfeldes des Bedienfelds.

2. Einrichtung nach Anspruch 1,
dadurch **gekennzeichnet**, daß zur Anwahl des jeweiligen Auswahlfeldes eine sog. Computer-Maus oder eine Rollkugel vorgesehen ist.

3. Einrichtung nach Anspruch 1,
dadurch **gekennzeichnet**, daß die Anwahl des jeweiligen Auswahlfeldes über die Tastatur und insbesondere die Cursor-Tasten erfolgt.

4. Einrichtung nach Anspruch 1,
dadurch **gekennzeichnet**, daß zur Anwahl des jeweiligen Auswahlfeldes der Bildschirm als sog. Touch-Screen ausgebildet ist.

5. Einrichtung nach einem der Ansprüche 1 bis 4,
dadurch **gekennzeichnet**, daß die Schnittstelle eine serielle Schnittstelle und insbesondere eine RS 232 C- oder RS 422-Schnittstelle ist.

6. Einrichtung nach einem der Ansprüche 1 bis 5,
dadurch **gekennzeichnet**, daß die von den einzelnen Geräten gelieferten Daten in ein Patienten-Stammdaten-und/oder ein Abrechnungsprogramm übernehmbar sind, das zusätzlich oder anstelle des Steuerungsprogramm ausführbar ist.

## Claims

1. Apparatus for the examination and/or treatment of the eyes with - at least one eye examination or treatment instrument such as a phoropter or a refractometer and, where necessary, auxiliary instruments such as a focimeter,
- a computer where the data determined by the eye examination instrument or instruments and, where used, the existing auxiliary instruments can be entered and wherein
- the instrument or instruments are connected to the computer via standard interfaces, and where
- the computer is designed in such a way that it not only receives data from the instrument or instruments, but also possesses a control program with which it controls the function of the instrument or instruments connected to it, **characterised** by the following features: - the computer possesses means which are designed in such a way that on the screen of the computer an operating field of the instrument or instruments being used in each case is displayed as a graphal interface where the selection fields of the operating field visually resemble the operating elements of the eye examination instrument or instruments of a conventional type connected to the computer, and
- the computer possesses further means for the control of the function of the instrument or instruments by the operating person by selection of the relevant selection field of the operating field.

2. Apparatus in accordance with claim 1, **characterised** in that a so-called computer mouse or trackerball is provided for the selection of the relevant selection field.

3. Apparatus in accordance with claim 1, **characterised** in that the selection of the relevant selection field is performed using the keyboard and in particular the cursor keys.

4. Apparatus in accordance with claim 1, **characterised** in that the screen is designed as a so-called touch-screen for the selection of the relevant selection field.

5. Apparatus in accordance with any of claims 1 to 4, **characterised** in that the interface is a serial interface and in particular is an RS 232 C or and RS 422 interface.

6. Apparatus in accordance with any of claims 1 to 5, **characterised** in that the data supplied from the individual instruments can be taken over into a master patient data program and/or an invoicing program which can be executed in addition to or in place of the control program.

## Revendications

1. Appareil pour l'examen et/ou le traitement des yeux, comportant
- au moins un appareil d'examen ou de traitement des yeux comme un phoromètre ou un réfractomètre ainsi qu'éventuellement des appareils auxiliaires, comme un organe de mesure de l'indice de distorsion pariétale,
- un ordinateur dans lequel peuvent entrer les données émises par le ou les appareils de traitement des yeux ainsi que des appareils auxiliaires éventuellement existants,
dans lesquels
- le ou les appareils sont réunis avec l'ordinateur par l'intermédiaire d'interfaces standard, et dans lequel
- l'ordinateur est conçu de façon que non seulement il reçoit les données provenant du ou des appareils, mais présente également un programme de commande avec lequel il commande le fonctionnement du ou des appareils qui lui sont reliés,
caractérisé par les attributs suivants:
- l'ordinateur présente des moyens qui sont conçus de façon que, sur l'écran de l'ordinateur, soit représenté comme surface graphique pour l'utilisateur, un panneau de service de ou des appareils respectivement utilisés, dont les zones de choix correspondent optiquement aux éléments de service du ou des appareils d'examen des yeux, de type traditionnel, reliés à l'ordinateur et
- l'ordinateur présente d'autres moyens pour la commande du fonctionnement du ou des appareils par l'opérateur de service par choix de la zone de choix respective du panneau de service.

2. Appareil selon la revendication 1,
caractérisé par le fait que pour choisir la zone de choix respective est prévue ce que l'on appelle une souris d'ordinateur ou une bille déroulante.

3. Appareil selon la revendication 1,
caractérisé par le fait que le choix de la zone de choix respective se fait par l'intermédiaire du clavier et en particulier des touches du curseur.

4. Appareil selon la revendication 1,
caractérisé par le fait que pour le choix de la zone de choix respective l'écran est conçu sous forme d'un écran dit tactile.

5. Appareil selon l'une des revendications 1 à 4,
caractérisé par le fait que l'interface est une interface série et en particulier une interface RS 232 C - ou RS 422.

6. Appareil selon l'une des revendications 1 à 5,
caractérisé par le fait que les données fournies par les différents appareils peuvent être transmises dans un programme d'établissement d'un fichier permanent du patient et/ou dans un programme de calcul qui peut s'exécuter en plus ou à la place du programme de commande.
